Europäisches Patentamt

European Patent Office

Office européen des brevets

(11) Publication number: **0 309 710**
**A1**

# EUROPEAN PATENT APPLICATION

(21) Application number: **88113217.9**

(22) Date of filing: **13.08.88**

(51) Int. Cl.⁴: **A61M 1/14 , A61M 1/34 , F24H 1/00**

(30) Priority: **17.09.87 SE 8703598**

(43) Date of publication of application:
**05.04.89 Bulletin 89/14**

(84) Designated Contracting States:
**BE CH DE ES FR GB IT LI NL**

(71) Applicant: **GAMBRO AB**
**Post Box 10101**
**S-220 10 Lund(SE)**

(72) Inventor: **Claren, Jan**
**Protokollgränden 38**
**S-222 47 Lund(SE)**
Inventor: **Grebius, Staffan**
**Adelgatan 13**
**S-223 50 Lund(SE)**

(74) Representative: **Boberg, Nils Gunnar Erik**
**Gambro AB Patent Department Box 10101**
**S-220 10 Lund(SE)**

(54) **Heating arrangement.**

(57) A heating arrangement comprising an inner rigid heating cartridge (1) and an outer envelope (2) with a space (14) for the heated medium, in particular a liquid, provided in between, together with inlet and outlet (3,4) for the heated medium connected to the envelope (2).

In accordance with the invention a flexible envelope (2) is used at the same time as the rigidity and tightness of the arrangement are achieved wholly or partly in that the envelope (2) is slipped over the heating cartridge (1) with an appropriate press fit.

Fig. 1

## HEATING ARRANGEMENT

### TECHNICAL FIELD

The present invention relates to a heating arrangement comprising an inner rigid heating cartridge and an outer envelope with a space for the heated medium, in particular a liquid, provided in between, together with inlet and outlet for the heated medium connected to the envelope.

The arrangement in accordance with the invention is intended in particular for the heating of dialysis fluid in connection with haemodialysis. Naturally though it can also be used for the heating of other liquids, e g replacement fluid in connection with haemofiltration or haemodiafiltration.

### BACKGROUND ART

Known arrangements of the abovementioned type suffer from the disadvantage that they are either simple and inexpensive but not very effective, or complicated and expensive. Special problems are also encountered, if an accurate fit between the heating cartridge used and the outer envelope is desired. Further problems of fitting arise, if it is desired to extend the path for the heated liquid through some special duct system.

The exact position regarding the prior art is not fully known to the applicant. The prior art known to the applicant, which comes closest to the invention described in the following, is evident from EP-B-58 303, which describes how a motor is cooled by means of a surrounding cooling jacket, that is to say a reverse technique in relation to heating.

### DISCLOSURE OF INVENTION

The abovementioned fitting problem is solved through the invention by virtue of the envelope being made flexible, and the rigidity and tightness being achieved wholly or partly in that the envelope is slipped over the heat cartridge with an appropriate press fit. An advantage gained at the same time is that the arrangement will be especially sutiable for the heating of medical fluids. The envelope, for example, can be made easily exchangeable, so that only the heating cartridge needs to be cleaned and sterilized between instances of application. Moreover, no glue or other bonding agent has to be applied, which otherwise would be liable to act upon the fluid treated. The envelope, moreover, can be made very cheaply and is particularly suitable. therefor, for single usage.

The said inlet and outlet are formed preferably integrally with the envelope. In this way the design is simplified further and made still less expensive.

In a preferred embodiment of the invention the arrangement is provided with fixing devices formed integrally with the envelope for the attachment of the arrangement to a wall. These fixing devices may be constituted, for example, of studs provided with thickened portions adapted so that owing to their elasticity they can be pressed through narrower holes into the said wall. Consequently. the design is simplified further and made still less expensive.

A particularly simple design is obtained if the envelope is given the form of a tube open at least at one of its ends. In a preferred embodiment of the subject of the invention the envelope is in the form of a tube open at both its ends, the heating cartridge being inserted from the one end and a plug being inserted with a press fit from the other end. The said plug may be provided with holes for the introduction of heat-sensing elements into the space between the heating cartridge and the plug. In this way the effectiveness of the arrangement can be easily checked and controlled.

Appropriately the envelope is provided on its inside with sealing grooves, for extending the path of flow between the said inlet and outlet.

Also the envelope itself can be provided with holes for introducing heat-sensing elements at suitable points into the space between the heating cartridge and the envelope. These holes, like the abovementioned holes, may be given a dimension which is such, that the heat-sensing elements too are introduced with a press fit. Alternatively the said elements may be set into the respective holes. The moulding compound in this case either has to be prevented from making contact with the heated fluid or it must be chosen so that it does not affect the same.

In a preferred embodiment of the object of the invention the said inlet and outlet are in the form of nipples formed integrally with the envelope, into which the connecting ducts are intended to be introduced, preferably with a press fit. Here too glue and other materials which might affect the heated liquid are avoided.

In cases where a press fit is not sufficient, e g when working with a liquid under pressure. the nipples may be provided with an internal flange intended to grip over a thickening on the associated connecting duct or a lock ring slipped onto this duct.

Further safety is achieved if the arrangement is provided with one or more rigid flanged rings capable of being slipped over the connecting ducts, intended, preferably with a press fit, to enclose the respective nipple so as to prevent expansion of the same and thereby a detachment of the connected duct.

The invention also relates to a coupling, intended primarily to be used in a system comprising an arrangement of the abovementioned type, characterized by one or more nipples into which connecting ducts are intended to be introduced, preferably with a press fit. These nipples too are provided appropriately with an internal flange intended to grip over a thickening on the associated connecting duct or a lock ring of the abovementioned type, at the same time as they are enclosed by rigid, flanged rings.

## BRIEF DESCRIPTIONS OF THE DRAWINGS

Fig 1 shows a longitudinal section through an arrangement in accordance with the invention with connecting ducts.

Fig 2 and Fig 3 show the arrangement seen from underneath and from above respectively. Naturally, though, it may also be used, for example, in horizontal position or in any arbitrary position.

Fig 4 shows an alternative design of a connecting nipple, intended for the arrangement in accordance with the invention.

Fig 5 shows an enlarged view of a fixing device, by means of which the arrangement in accordance with the invention can be attached to a wall.

Fig 6 and 7 finally show two different couplings which are intended in particular to be used in a system comprising an arrangement of the abovementioned type. At the same time these figures are intended to show further possible variations of the connecting nipples shown in fig 1-4.

## PREFERRED EMBODIMENTS OF THE INVENTION

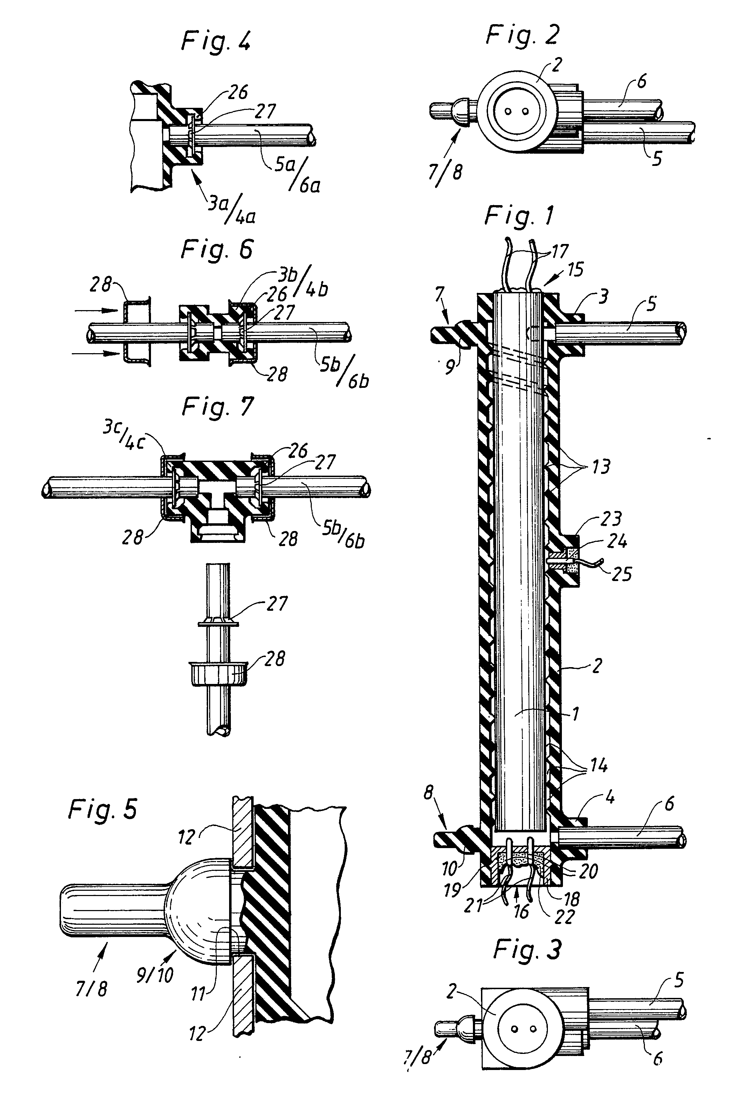

In fig 1 is thus shown a preferred embodiment of a heating arrangement in accordance with the invention. Numeral 1 designates an inner, rigid heating cartridge, whose outer surface may consist of steel, porcelain, ceramic or the like, preferably bright steel. The heating cartridge 1 is surrounded by an outer flexiable envelope 2, which preferably is manufactured from silicon rubber, but which naturally may also consist of other flexible material. Numerals 3 and 4 designate two nipples moulded integrally with the envelope, intended to serve as inlet and outlet for the heated medium. Into the nipples 3 and 4, therefore, connecting ducts 5 and 6 are introduced with a press fit. Opposite the respective nipples are shown studs 7 and 8 provided with thickened portions 9 and 10 serving as fixing devices. Illustrated on a larger scale in fig 5 the studs are intended to be pressed through holes 11 into a wall 12. This wall may be constituted, for example, of an inner wall or an outer wall in a monitor intended for the control of a dialysis.

On its inner surface the envelope 2 is provided with grooves 13 preferably arranged in helical form which between them create a helical channel 14. In this manner the path of flow between inlet and outlet 3 and 4 is considerably extended.

As is evident from the figure, the envelope is made initially with open ends 15 and 16. Through the opening 15 the heating cartridge is introduced into the envelope 2 with a press fit so, that a sealing connection is obtained on the one hand with the top end of the envelope, on the other hand with the helical groove 13. With the help of leads 17 the heating cartridge is connected to a suitable power source and appropriate controls. Into the other end 16 of the envelope is inserted with a press fit a pluglike wall 18. The seal obtained through the press fit can be further improved by means of a sealing groove 19. Through the end wall 18 project two heat-sensing elements 20 into the space between the end wall and the heating cartridge. These elements are connected by means of leads 21 to the abovementioned controls for the heating cartridge. In the example shown the elements 20 are set into a moulding compound 22. Alternatively they can be inserted with a press fit into narrower holes in the end wall 18.

Approximately in the centre of the envelope is a nipple 23 wherein a further heat-sensing element 24 is inserted into the flow path. This element too is connected by means of a lead 25 to the said controls. If the nipple 3 here serves as an inlet, the element 24 helps to bring about a preliminary sensing of the temperature in order to facilitate obtaining the desired end temperature at the outlet 4. This end temperature is finely adjusted and monitored by means of the elements 20.

In fig 4 is shown an alternative design of the nipples 3 and 4 which here have been marked 3a/4a and provided with an inwardly facing flange 26. This flange is intended to grip over a retaining ring 27 slipped onto the tubing 5a/6a.

In fig 6 and 7 are shown two couplings which are intended to be used preferably in a system comprising a heating cartridge of the abovementioned type. At the same time these figures illustrate how the nipples 3 and 4 can be further modified in that rigid flanged rings 28 are slipped onto the tubings, here marked 5b/6b, which, preferably with a press fit, enclose the respective "nipples" here marked 3b/4b and 3c/4c respectively.

Naturally the invention is not limited solely to the components described above and shown on the drawing, but may be varied within the scope of the following claims. For example, it also may be applied to a tubular heating cartridge. The medium intended for heating in this case is made to flow in the first place through the tubular cartridge in order to flow afterwards through a duct corresponding to the duct 14.

## Claims

1. A heating arrangement comprising an inner rigid heating cartridge (1) and an outer envelope (2) with a space (14) for the heated medium, in particular a liquid, provided in between, together with inlet and outlet (3,4) for the heated medium connected to the envelope, **characterized** in that the envelope (2) is flexible and that the rigidity and tightness of the arrangement are achieved wholly or partly in that the envelope (2) is slipped over the heating cartridge (1) with an appropriate press fit.

2. An arrangement in accordance with claim 1, **characterized** in that the said inlet and outlet (3,4) are formed integrally with the envelope.

3. An arrangement in accordance with claim 1, **characterized** by fixing devices (7,8) formed integrally with the envelope (2) for the attachment of the arrangement to a wall (12).

4. An arrangement in accordance with claim 3, **characterized** in that the said fixing devices (7,8) are constituted of studs provided with thickened portions (9,10), adapted so that owing to their elasticity they can be pressed through narrower holes in the said wall (12).

5. An arrangement in accordance with anyone of the preceding claims, **characterized** in that the envelope (2) is in the form of a tube open at least on one of its ends.

6. An arrangement in accordance with anyone of the preceding claims, **characterized** in that the envelope (2) is in the form of a tube open at both its ends, the heating cartridge (1) being inserted from the one end (15) and a plug (18) being inserted with a press fit from the other end (16).

7. An arrangement in accordance with claim 6, **characterized** in that the said plug (18) is provided with holes for the introduction of heat-sensing elements (2) into the space between the heating cartridge (1) and the plug (18).

8. An arrangement in accordance with anyone of the preceding claims, **characterized** in that the envelope (2) is provided on its inside with sealing grooves (13) located against the cartridge (1) for the extension of the flow path between inlet and outlet (3,4).

9. An arrangement in accordance with anyone of the preceding claims, **characterized** in that the envelope (2) itself is provided with holes for the introduction of heat-sensing elements (24) into the space (14) between the heat cartridge (1) and the envelope (2).

10. An arrangement in accordance with anyone of the preceding claims, **characterized** in that the said inlet and outlet (3,4) are in the form of nipples formed integrally with the envelope, wherein the connecting ducts are intended to be introduced, preferably with a press fit.

11. An arrangement in accordance with claim 10, **characterized** in that said nipples (3,4) are provided with an internal flange (26) intended to grip over a thickening on the associated connecting duct.

12. An arrangement in accordance with claim 10 and/or 11, **characterized** by one or more rigid flanged rings (28) which can be slipped over the said connecting ducts, intended to enclose, preferably with a press fit, the respective nipple (3b,4b; 3c,4c) so as to prevent expansion of the same and thereby a detachment of the connecting duct (5b,6b).

13. A coupling intended primarily to be used in a system comprising an arrangement in accordance with anyone of the preceding claims, **characterized** by one or more nipples (3b,4b; 3c,4c) into which the connecting ducts (5b,6b) are intended to be introduced, preferably with a pressure fit.

14. A coupling in accordance with claim 13, **characterized** in that the said nipples (3b,4b; 3c,4c) are provided in an internal flange (26), intended to grip over a thickening (27) on the associated connecting duct (5b,6b).

15. A coupling in accordance with claim 14, **characterized** by one or more rigid, flanged rings (28) capable of being slipped over the said connecting ducts (5b,6b) intended to enclose, preferably with a press fit, the respective nipple (3b,4b; 3c,4c) so as to prevent an expansion of the same and thereby a detachment of the connected ducts (5b,6b).

*Fig. 4*

*Fig. 2*

*Fig. 1*

*Fig. 6*

*Fig. 7*

*Fig. 5*

*Fig. 3*

European Patent Office

## EUROPEAN SEARCH REPORT

Application number

| | DOCUMENTS CONSIDERED TO BE RELEVANT | | EP 88113217.9 |
|---|---|---|---|

| Category | Citation of document with indication, where appropriate, of relevant passages | Relevant to claim | CLASSIFICATION OF THE APPLICATION (Int. Cl.4) |
|---|---|---|---|
| Y | US - A - 3 443 060 (E.M.SMITH)<br><br>* Totality; especially fig. 1,3,4-6,9,10; abstract; column 2, line 51 - column 3, line 32 *<br><br>-- | 1,2,5,<br>8,10,<br>13 | A 61 M 1/14<br><br>A 61 M 1/34<br><br>F 24 H 1/00 |
| D,Y | EP - A1 - 0 058 303 (GAMBRO AB)<br><br>* Fig. 1,3; page 3, lines 15-19; page 5, lines 14-27 *<br><br>---- | 1,2,5,<br>8,10,<br>13 | |
| | | | **TECHNICAL FIELDS SEARCHED (Int. Cl.4)**<br><br>A 61 M 1/00<br><br>A 61 M 5/00<br><br>F 24 H 1/00 |

The present search report has been drawn up for all claims

| Place of search | Date of completion of the search | Examiner |
|---|---|---|
| VIENNA | 20-01-1989 | LUDWIG |